# EUROPEAN PATENT APPLICATION

(11) **EP 4 592 668 A1**
(43) Date of publication of application: **30.07.2025**
(21) Application number: 24834222.2
(22) Date of filing: 27.11.2024
(51) Int. Cl.: G01N 17/00, G01N 33/00

(54) **CHAMBER SYSTEM**

(30) Priority: 08.12.2023 KR 20230177285; 22.11.2024 KR 20240167903
(71) Applicant: Industry Academic Cooperation Foundation of Yeungnam University, Gyeongsan-si, Gyeongsangbuk-do 38541 (KR)
(72) Inventor: BYEON, Jeong Hoon, Gyeongsan-si, Gyeongsangbuk-do 38541 (KR)
(74) Representative: Murgitroyd & Company
(86) International application number: PCT/KR2024/018981
(87) International publication number: WO 2025/121771

(57) **Abstract**

The present disclosure provides a chamber system useful for studying the effect of a pollutant, particularly, an aerosol type pollutant, on a living body.

## Description

### BACKGROUND

### 1. Field of the Invention

The present disclosure relates to a chamber system, and more specifically, to a chamber system capable of analyzing a biological correlation between an aerosol and a living body.

### 2. Discussion of Related Art

In modern society where technology is highly developed, interest in environmental protection and ecosystem restoration is increasing.

Studies on the effects of pollutants on living bodies have been actively conducted in order to protect the environment and restore the ecosystem, but apparatuses useful for such studies have not been developed yet.

### SUMMARY OF THE INVENTION

The present disclosure is directed to providing a chamber system useful for studying the effect of a pollutant, particularly, an aerosol type pollutant, on a living body.

According to an aspect of the present invention, there is provided a chamber system including a chamber having an accommodation space for accommodating a living body, a supply part which supplies a sample aerosol to the living body in the accommodation space, a monitoring part which monitors changes in motion or image of the living body before and after the sample aerosol is supplied to the living body, and an analysis part which analyzes a biological correlation between the sample aerosol and the living body on the basis of a monitoring result.

In addition, the sample aerosol may include at least one of solid particles, liquid particles, and composite particles in which liquid particles and solid particles are bonded.

In addition, the solid particles may include metal particles or non-metal particles.

In addition, the liquid particles may include an organic compound, an inorganic compound, or a mixture thereof.

In addition, the supply part may include a manufacturing part which manufactures the sample aerosol.

In addition, the chamber system may further include a pollutant supply part provided to supply a gaseous pollutant to the living body in the accommodation space.

In addition, the gaseous pollutant may include one or more selected from the group consisting of carbon monoxide, carbon dioxide, formaldehyde, nitrogen dioxide, sulfur dioxide, hydrogen sulfide, a volatile organic compound, aromatic hydrocarbon, methylene chloride, ethylene chloride, ammonia, ozone, naphthalene, and radon.

In addition, the chamber system may include a sensor which measures the quality of air in the accommodation space.

In addition, the chamber may include a ventilation fan which supplies external air to the accommodation space and discharges internal air in the accommodation space to an outside and a control unit which controls operation of at least one of the ventilation fan, the supply part, and the pollutant supply part on the basis of a measurement result of the sensor.

In addition, the control unit may have a first mode in which at least one of the ventilation fan, the supply part, and the pollutant supply part turns on and operates when the measurement result of the sensor is greater than a set reference value and a second mode in which at least one of the ventilation fan and the pollutant supply part turns off and does not operate when the measurement result of the sensor is not greater than the set reference value.

In addition, the chamber may include a collection part which collects a sample candidate group including at least one of the sample aerosol and the gaseous pollutant supplied to the accommodation space.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a block diagram illustrating a chamber system according to one embodiment of the present disclosure;
FIGS. 2 and 3 are views for helping understanding of the chamber system through which the effect of a sample aerosol on an animal is studied; and
FIGS. 4 and 5 are views for helping understanding of the chamber system through which the effect of a sample aerosol on a plant is studied.

### DETAILED DESCRIPTION OF EXEMPLARY EMBODIMENTS

The present disclosure relates to a chamber system.

The chamber system according to the present disclosure may be an apparatus useful for studying the effect of pollutants on living bodies.

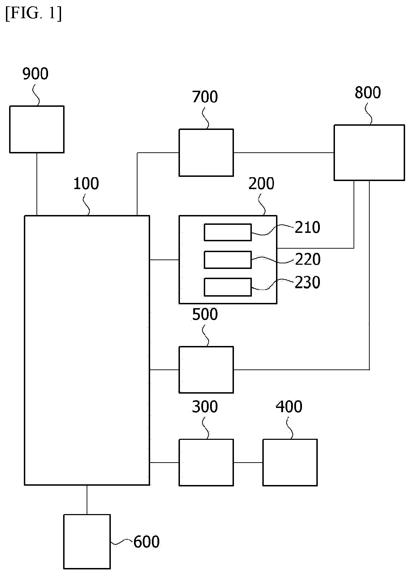

FIG. 1 is a block diagram illustrating the chamber system according to one embodiment of the present disclosure.

The chamber system according to the present disclosure includes a chamber 100, a supply part 200, a monitoring part 300, and an analysis part 400.

The chamber 100 has an accommodation space for accommodating a living body. A type of living body may not be limited to bacteria, insects, animals, plants, etc., and may also correspond to human skin tissue or organ tissue which may respond to external stimuli. A proper size of the chamber 100 may be designed based on a size of the living body.

In addition, the chamber 100 may be manufactured of a material with low reactivity to a pollutant or coated with the material. The material may be, for example, Teflon.

The chamber 100 may include a heater for supplying heat into the accommodation space, a dehumidifying apparatus for removing moisture in the accommodation space, and a temperature and humidity adjustment part for adjusting a temperature and a humidity in the accommodation space by controlling operations of the heater and the dehumidifying apparatus. In addition, the chamber 100 may additionally include a lighting apparatus.

In addition, an alarm apparatus for detecting a situation of an excessive temperature change, gas leak, etc., and notifying the situation to the outside may be provided in the chamber 100.

In addition, the chamber 100 may be formed of a transparent material or may have a transparent window to allow easy observation of the accommodation space from the outside.

The supply part 200 supplies a sample aerosol to the living body in the accommodation space. The supply part 200 is a sample aerosol supply part. In the present invention, the sample aerosol is solid particles, liquid particles (also referred to as droplets), or composite particles in which liquid particles and solid particles are bonded, which are floating in air.

In the present invention, the sample aerosol may be an aerosol generated from a daily environment (for example, an aerosol generated during cooking) to a specific environment (for example, an aerosol generated during welding) or an aerosol manufactured by simulating them.

The supply part 200 may supply the sample aerosol to the living body in the accommodation space using a mechanical atomizer, a nebulizer, an impinger, a mechanical spray, an ultrasonic spray, an electrospray, or a gas injection dust dispenser.

The supply part 200 may include a supply amount adjusting part which adjusts a supply amount of the sample aerosol. The supply amount adjusting part may adjust a concentration of the sample aerosol in the accommodation space by adjusting the supply amount of the sample aerosol.

In addition, the supply part 200 may adjust a supply frequency of the sample aerosol. For example, the supply part 200 may supply the sample aerosol a predetermined number of times for a predetermined time. As the supply frequency of the sample aerosol is adjusted, the analysis part 400 may be assisted in checking functional or dysfunctional health effect of the living body according to exposure of sample aerosol in the acute, subchronic, and chronic types.

The monitoring part 300 monitors changes in motion or image of the living body before and after the sample aerosol is supplied. For example, the monitoring part 300 may include a camera which captures an image of the living body and a storage part which stores the image captured by the camera. Any one of various types of cameras such as a high-resolution camera and an infrared camera may be used as the camera. Particularly, the infrared camera may check a change in the body temperature of the living body.

In addition, the monitoring part 300 may include various markers which detect changes in movement, heart rate, respiratory rate, body temperature, and the like of the living body, and the markers may be attached to the living body.

The monitoring part 300 may monitor changes in behavior, body temperature, shape of a skin portion, or color of the living body before and after the living body is exposed to the sample aerosol.

For example, the monitoring part 300 may monitor a change in behavior of the living body using a heat map. Specifically, the monitoring part 300 may generate a virtual map by converting the chamber into a two-dimensional (2D) or three-dimensional (3D) map and monitor a change in behavior of the living body by displaying a moving path, a staying time at a specific position, and the like of the living body on the generated virtual map.

For example, the monitoring part 300 may operate in conjunction with a cloud-based system such that a user remotely accesses and analyzes data. Accordingly, the user can check the progress of an experiment anytime, anywhere, and take immediate action as necessary.

The analysis part 400 analyzes a biological correlation between the sample aerosol and the living body based on a monitoring result. The biological correlation may include various analysis items, such as a physiological response, a genetic change, and a biochemical response.

The analysis part 400 may analyze a pattern of a change in motion or image of the living body before and after exposure to the sample aerosol and precisely analyze the effect of a particle size, a type, chemical properties, an exposure time, and the like of the sample aerosol on the behavior, growth, health state, and the like of the living body from the pattern analysis.

In addition, the analysis part 400 may analyze the functional or dysfunctional health effect of the sample aerosol.

For example, the analysis part 400 may analyze the effect of acute, sub-chronic, and chronic types of diseases of the living body according to the exposure of the sample aerosol. Examples of such diseases may include respiratory diseases, cardiovascular diseases, cerebrovascular diseases, pulmonary diseases, endocrine diseases, blood diseases, liver diseases, obesity-related diseases, mental disorders, urinary diseases, reproductive diseases, neurological diseases, autoimmune diseases, genetic disorders, skin diseases, eye diseases, periodontal diseases, behavioral disorders, etc.

A deep-learning based learning algorithm may be applied to the analysis part 400 to perform the analysis.

In addition, the analysis part 400 may analyze the effect on the behavior, growth, health state, and the like of the living body according to a concentration of the sample aerosol.

In addition, the analysis part 400 may analyze the effect on the behavior, growth, health state, and the like of the living body according to a supply frequency of the sample aerosol.

In one embodiment, the sample aerosol may include at least one type of particle among solid particles, liquid particles, and composite particles that liquid particles and solid particles are bonded. The sample aerosol may be particles floating in the air.

The solid particles may include, for example, metal particles or non-metal particles. An example of the metal particles may be iron particles, aluminum particles, lead particles, etc., and an example of the non-metal particles may be silica particles, latex particles, carbon-based particles, etc.

The liquid particles may include, for example, an organic compound, an inorganic compound, or a mixture thereof. Examples of the organic compound may be alcohol, ketone, ester, etc., and examples of the inorganic compound may be sodium hydroxide, potassium hydroxide, sulfuric acid, etc.

As an example, the liquid particles may include various natural substances found in daily life or simulants thereof. In this case, the simulants are substances artificially manufactured to be similar to the natural substances. For example, the liquid particles may include oil droplets such as oil mist generated during cooking, volatile organic compound droplets formed by a volatile organic compound being evaporated and aggregated, and water-soluble droplets formed by water-soluble substances, such as nitrogen oxides, sulfur oxides, etc., being dissolved in moisture in the air and aggregated.

In addition, the composite particles may have a core-shell structure in which the liquid particles surround solid particles.

In one specific embodiment, the supply part 200 may include a first supply part 210 for supplying a first sample aerosol including solid particles, a second supply part 220 for supplying a second sample aerosol including liquid particles, and a third supply part 230 for supplying a third sample aerosol including composite particles.

In one embodiment, the supply part 200 may include a manufacturing part which manufactures the sample aerosol. The manufacturing part may be an apparatus separated from the supply part 200 or integrally coupled with the supply part 200. When the manufacturing part and the supply part 200 are integrally coupled, the sample aerosol is manufactured by one apparatus, and the manufactured sample aerosol may be supplied to the living body in the accommodation space.

The first supply part 210 may include a first manufacturing part 210 which manufactures a first sample aerosol including solid particles, the second supply part 220 may include a second manufacturing part 220 which manufactures a second sample aerosol including liquid particles, and the third supply part 230 may include a third manufacturing part 230 which manufactures a third sample aerosol including composite particles. The first manufacturing part 210 may include a spark ablation apparatus, the second manufacturing part 220 may include an atomizer or nebulizer, and the third manufacturing part 230 may include a spark ablation apparatus, an atomizer, or a nebulizer.

In the present invention, each of the first to third supply parts and the first to third manufacturing parts may be understood as an integrally coupled apparatus and marked with the same reference numeral, but is not limited thereto, and the first to third supply parts and the first to third manufacturing parts may be separated as different apparatuses.

In addition, the first to third manufacturing parts 210, 220, and 230 may include gas supply apparatuses for supplying gas such as air, and particles manufactured by the first to third manufacturing parts 210, 220, and 230 may be manufactured in the form of an aerosol by being mixed with the gas supplied from the gas supply apparatuses.

The solid particles manufactured by the third manufacturing part 230 through spark ablation may be bonded with the liquid particles generated by the atomizer or the nebulizer and formed as composite particles.

In addition, the third manufacturing part 230 may include a photoirradiation apparatus. The photoirradiation apparatus may emit light to the composite particles, increase a bonding force between components of the composite particles by the photoirradiation, and induce the composite particles to be stiffened.

The spark ablation apparatus may manufacture metal nanoparticles from a metal electrode through spark discharge. In this case, the "spark discharge" is a high frequency discharge method performed in a kV-mA mode at a normal pressure. In addition, in the present disclosure, the term "nano" may be a size in nanometers (nm), and may mean, for example, a size of 1 to 1,000 nm, but is not limited thereto. In addition, in the present disclosure, the term "nanoparticles" may be particles having an average diameter in nanometers (nm), and may be particles having an average diameter of, for example, 1 to 1,000 nm, but is not limited thereto.

The spark ablation apparatus may include, for example, a discharge part, an alternating current (AC) power supply part, and an AC power control part.

The discharge part is a part which generates metal nanoparticles through spark discharge and includes a pair of metal electrodes disposed to be spaced a predetermined distance from each other, and the pair of metal electrodes are disposed to be spaced apart from each other to form a gap. For example, in the discharge part, the metal nanoparticles may be generated by a high temperature locally occurring between the metal electrodes by spark discharge.

A metal material for forming the metal electrode is not specifically limited as long as the metal material is a conductive material, and for example, the metal electrodes may include one or more selected from the group consisting of aluminum, antimony, tin, bismuth, carbon nanotubes, cerium, copper, cobalt, fullerene, polyhedral oligomeric silsesquioxane (POSS), graphene, iron, magnesium, manganese, lead, gold, silver, nickel, silicon, titanium, yttrium, zinc, and zirconium.

In addition, the metal nanoparticles generated from the metal electrodes may react with oxygen gas passing between the metal electrodes and may be changed to an oxide thereof. For example, types of metal nanoparticles generated from the gap between the metal electrodes may include one or more selected from the group consisting of aluminum, antimony, tin, bismuth, carbon nanotubes, cerium, copper, cobalt, fullerene, polyhedral oligomeric silsesquioxane (POSS), graphene, iron, magnesium, manganese, lead, gold, silver, nickel, silicon, titanium, yttrium, zinc, zirconium, and an oxide thereof.

The AC power supply part may be a part which supplies AC power or electric power to the metal electrodes. The AC power supply part may apply a pulse signal for the AC power to the metal electrodes. When the AC power is applied as described above, since an additional circuit for changing a frequency is not needed unlike direct current (DC) power, there is an advantage that the frequency can be easily controlled.

A frequency and voltage of the AC power generated by the AC power supply part may be controlled by the AC power control part. The AC power control part serves to control the frequency and voltage of the AC power applied to the metal electrodes to be within a predetermined range and uniformly supply an AC current to the metal electrodes.

The AC power control part may control the frequency of the AC power to be 20 kHz or more, for example, 30 kHz or more or 40 kHz or more. An upper limit of the frequency is not specifically limited, and for example, may be 1 MHz or less.

In addition, the AC power control part may control the voltage of the AC power to be 2.2 to 5.0 kV, for example, 2.5 kV to 4.5 kV, 2.2 kV to 3.5 kV, 3.0 kV to 4.0 kV, or 3.5 kV to 5.0 kV, but the present invention is not limited thereto.

Not only a particle size of metal nanoparticles generated from the metal electrodes may be maintained in nanometers but an amount of the metal nanoparticles manufactured per unit time may also be maximized by controlling the frequency and the voltage of the AC power applied to the metal electrodes to be within the above-described ranges even when the inorganic particles are manufactured using the spark discharge method instead of an arc discharge method.

In addition, the spark ablation apparatus may include a DC power part instead of the AC power supply part.

In one embodiment, the first to third the supply parts 210, 220, and 230 (or the first to third manufacturing parts) may be connected to each other in series or in parallel.

For example, the serial connection may be applied to composite sample aerosols manufactured in the first to third the supply parts 210, 220, and 230 (or first to third manufacturing parts) in a layer-by-layer form, and the parallel connection may be applied to randomly aggregate sample aerosols manufactured by the supply parts.

The chamber system according to the present invention may manufacture a sample aerosol by simulating an aerosol generated from a daily environment (for example, an aerosol generated during cooking) to a specific environment (for example, an aerosol generated during welding) using the first to third manufacturing parts 210, 220, and 230.

In one embodiment, a pollutant supply part 500 provided to supply a gaseous pollutant to the living body in the accommodation space may be additionally included.

The analysis part 400 may analyze the effect of the gaseous pollutant on the behavior, growth, health state, and the like of the living body from changes in motion or image of the living body before and after the gaseous pollutant is supplied thereto. In addition, the analysis part 400 may analyze the effect of the sample aerosol and the gaseous pollutant on the behavior, growth, health state, and the like of the living body when the sample aerosol and the gaseous pollutant are supplied together.

For example, the gaseous pollutant may include one or more selected from the group consisting of carbon dioxide, formaldehyde, nitrogen dioxide, sulfur dioxide, hydrogen sulfide, a volatile organic compound, aromatic hydrocarbon, methylene chloride, ethylene chloride, ammonia, ozone, naphthalene, and radon, but are not limited thereto, and may include various known gaseous pollutants.

Particularly, carbon monoxide or nitrogen dioxide is suitable for studying air pollution in a high-traffic city, and formaldehyde or volatile organic compound may be mainly used for studying indoor air quality. Radon may be used for studying exposure to the human body in the quality of indoor air including radon emitted from building materials and in air pollution including radon emitted from soil, etc.

In addition, the present invention may include a data storage part which stores a monitoring result and an analysis result as data. The user may understand a reaction of the living body exposed to the sample aerosol using the data stored in the data storage part and immediately take follow-up actions therefor.

In one specific embodiment, a bioaerosol supply part, which is provided to supply bioaerosol to the living body in the accommodation space, may be additionally included. The bioaerosol refers to viruses, bacteria, fungi, pollen, or the like floating in the air.

The bioaerosol supply part may supply bioaerosol using a vibration mesh spray to prevent damage to the bioaerosol.

In another embodiment, a sensor 600 which measures the quality of air in the accommodation space may be included. An example of the sensor 600 may be a temperature sensor, a humidity sensor, a carbon dioxide sensor, a fine dust sensor, a gas sensor, etc. A measurement result of the sensor 600 may be transmitted to the monitoring part 300 in real time. The user can check not only a change in motion or image of the living body but also a change in the quality of air in the accommodation space, and accordingly, users can be assisted in checking a correlation between the sample aerosol and the air quality.

In one specific embodiment, the chamber 100 may include a ventilation fan 700 which supplies external air to the accommodation space and discharges internal air of the accommodation space to the outside and a control unit 800 which controls operation of at least one of the ventilation fan 700, the supply part 200, and the pollutant supply part 500 on the basis of a measurement result of the sensor 600.

The chamber system according to the present invention may additionally include a filter part for purifying the internal air discharged to the outside by the ventilation fan 700. The filter part may include an air filter. The air filter may have a function which selectively removes specific gases. Since the performance of the air filter may be degraded according to a use time, an alarm part for notifying a filter replacement period may be provided.

For example, the control unit 800 may have a first mode in which at least one of the ventilation fan 700, the supply part 200, and the pollutant supply part 500 turns on and operates when a measurement result of the sensor 600 is greater than a set reference value and a second mode in which at least one of the ventilation fan 700, the supply part 200, and the pollutant supply part 500 turns off and does not operate when the measurement result of the sensor 600 is not greater than the set reference value.

The control unit 800 may adjust a rotational speed of the ventilation fan 700 in the first and second modes or adjust a supply amount of sample aerosol supplied from the supply part 200 and a supply amount of the gaseous pollutant supplied from the pollutant supply part 500. In the first mode, according to an extent by which the measurement result of the sensor 600 exceeds a set reference value, the rotational speed of the ventilation fan 700 may be increased or decreased, the supply amount of the gaseous pollutant may be increased or decreased, and the supply amount of the sample aerosol may be increased or decreased.

The control unit 800 may include a user interface so that the user may directly control operation of the ventilation fan 700. Accordingly, the user may adjust an environment of the accommodation space according to a study target thereof.

In one embodiment, the chamber 100 may include a collection part 900 for collecting a sample candidate group including at least one among a sample aerosol and a gaseous pollutant supplied to the accommodation space.

The collection part 900 may be installed in at least one of a front end and a rear end of the chamber 100. In addition, the collection part 900 may collect the sample candidate group in a gaseous state or liquid state.

Any of various known apparatuses usable for collecting gas or liquid may be used as the collection part 900 without limitation, and a sample of the collection part 900 may be a collection substrate, a gas bag sampling for collecting gas, a liquefaction apparatus for collecting liquid, etc.

The analysis part 400 may perform physical chemical and biological analyses of the sample aerosol and the gaseous pollutant using the sample candidate group collected in the collection part.

Hereinafter, the chamber system through which the effect of the sample aerosol on the living body may be studied will be easily described.

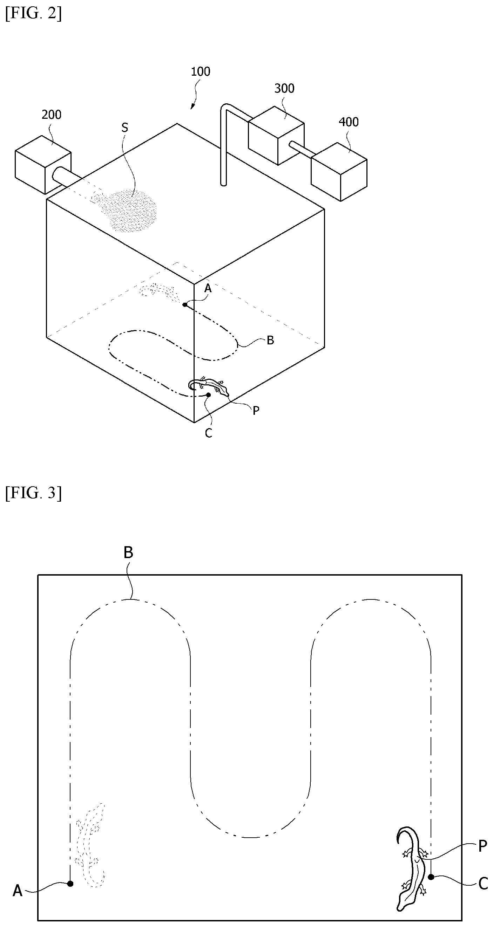

FIGS. 2 and 3 are views for helping understanding of the chamber system through which the effect of a sample aerosol on an animal is studied.

In one embodiment, a behavior pattern of a living animal P (for example, a lizard or rat) exposed to a sample aerosol was analyzed using the chamber system according to the present invention.

Referring to FIG. 2, the chamber 100 in which the living animal P was accommodated is prepared, and a sample aerosol S was supplied to the accommodation space of the chamber 100 by the supply part 200. In addition, in the monitoring part 300, the accommodation space of the chamber 100 was converted into a 2D map, and a start position A, a moving path B, and an arrival position C of the living animal P were marked on the converted 2D map as in FIG. 3.

In addition, not only a position of the living animal P but also a time for which the living animal P remained at a specific position was marked on the 2D map through the monitoring part 300. For example, a position at which the living animal P remained for a relatively long time was marked with a bright color, and a position at which the living animal P remained for a relatively short time is marked with a dark color.

In addition, the analysis part 400 stored a moving pattern of the living animal P and analyzed whether the sample aerosol S had toxicity on the basis of a tendency of the stored moving pattern. For example, the analysis part 400 analyzed that a sample aerosol causing a tendency of a similar moving pattern had a similar toxicity.

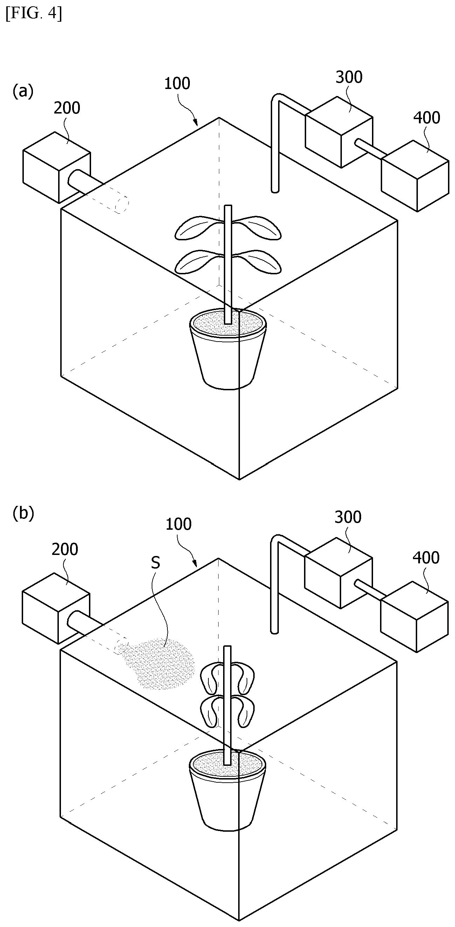

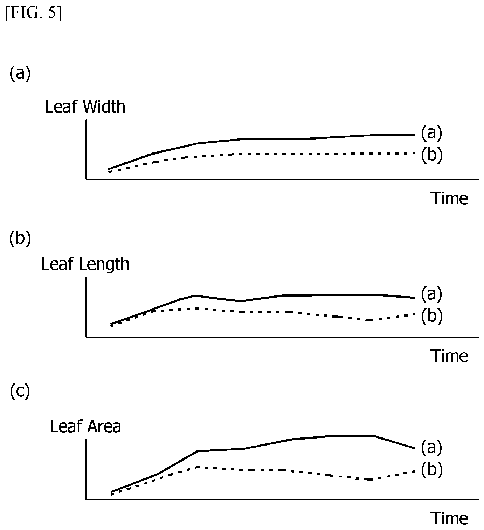

FIGS. 4 and 5 are views for helping understanding of the chamber system through which the effect of a sample aerosol on a plant is studied.

In another embodiment, a growth pattern of a plant exposed to a sample aerosol was analyzed using the chamber system according to the present invention.

FIG. 4A is a view illustrating the chamber system to which a sample aerosol is not supplied, FIG. 4B is a view illustrating the chamber system to which the sample aerosol is supplied, and FIG. 5 shows a result of analyzing growth patterns of plants grown in the chamber system of FIGS. 4A and 4B.

First, as in FIG. 4A, the chamber 100 in which the plant was accommodated was prepared as a control group, and a sample aerosol was not supplied to the chamber 100.

As in FIG. 4B, the chamber 100 in which the plant was accommodated was prepared as an experimental group, and a sample aerosol S was supplied to the accommodation space of the chamber 100 using the supply part 200. In addition, as in FIG. 5, changes in leaf length, leaf width, and leaf area according to time were monitored by the monitoring part 300.

The analysis part 400 analyzed that the supplied sample aerosol in FIG. 4B suppressed the growth of the plant from the result of FIG. 5. In addition, the analysis part 400 stored the result of FIG. 5 and analyzed whether the sample aerosol S had toxicity on the basis of a tendency of stored graphs.

For example, the analysis part 400 analyzed that a sample aerosol causing a similar graph tendency had a similar toxicity.

A chamber system according to the present disclosure is advantageous for studying the effect of an aerosol type pollutant on a living body.

The above-described exemplary embodiment of the present invention is disclosed to exemplify the present invention and may be variously changed, modified, and added to by those skilled in the art within the spirit and scope of the present invention, and such changes, modifications, and additions may fall within the scope of the appended claims.

## Claims

1. A chamber system comprising:
a chamber having an accommodation space for accommodating a living body;
a supply part which supplies a sample aerosol to the living body in the accommodation space;
a monitoring part which monitors changes in motion or image of the living body before and after the sample aerosol is supplied to the living body; and
an analysis part which analyzes a biological correlation between the sample aerosol and the living body on the basis of a monitoring result.

2. The chamber system of claim 1, wherein the sample aerosol includes at least one of:
solid particles;
liquid particles; and
composite particles in which liquid particles and solid particles are bonded.

3. The chamber system of claim 2, wherein the solid particles include metal particles or non-metal particles.

4. The chamber system of claim 2, wherein the liquid particles include an organic compound, an inorganic compound, or a mixture thereof.

5. The chamber system of claim 1, wherein the supply part includes a manufacturing part which manufactures the sample aerosol.

6. The chamber system of claim 1, further comprising a pollutant supply part provided to supply a gaseous pollutant to the living body in the accommodation space.

7. The chamber system of claim 6, wherein the gaseous pollutant includes one or more selected from the group consisting of carbon monoxide, carbon dioxide, formaldehyde, nitrogen dioxide, sulfur dioxide, hydrogen sulfide, a volatile organic compound, aromatic hydrocarbon, methylene chloride, ethylene chloride, ammonia, ozone, naphthalene, and radon.

8. The chamber system of claim 6, comprising a sensor which measures quality of air in the accommodation space.

9. The chamber system of claim 8, wherein the chamber includes:
a ventilation fan which supplies external air to the accommodation space and discharges internal air in the accommodation space to an outside; and
a control unit which controls operation of at least one of the ventilation fan, the supply part, and the pollutant supply part on the basis of a measurement result of the sensor.

10. The chamber system of claim 9, wherein the control unit has:
a first mode in which at least one of the ventilation fan, the supply part, and the pollutant supply part turns on and operates when the measurement result of the sensor is greater than a set reference value; and
a second mode in which at least one of the ventilation fan and the pollutant supply part turns off and does not operate when the measurement result of the sensor is not greater than the set reference value.

11. The chamber system of claim 7, wherein the chamber includes a collection part which collects a sample candidate group including at least one of the sample aerosol and the gaseous pollutant supplied to the accommodation space.
